(19) Europäisches Patentamt
European Patent Office
Office européen des brevets

(11) Numéro de publication: 0 143 018
B1

(12) # FASCICULE DE BREVET EUROPÉEN

(45) Date de publication du fascicule du brevet: **02.09.87**

(51) Int. Cl.⁴: **C 07 D 215/22** // C07D265/06, C07C125/03

(21) Numéro de dépôt: **84401863.0**

(22) Date de dépôt: **20.09.84**

(54) **Procédé de préparation de quinolinones-4.**

(30) Priorité: **22.09.83 FR 8315072**

(43) Date de publication de la demande: **29.05.85 Bulletin 85/22**

(45) Mention de la délivrance du brevet: **02.09.87 Bulletin 87/36**

(84) Etats contractants désignés: **AT BE CH DE FR GB IT LI LU NL SE**

(56) Documents cité:
**EP-A-0 056 763**
**FR-A-2 312 491**
**US-A-4 013 662**

**CHEMICAL ABSTRACTS, volume 51, colonne 16293h, (COLUMBUS, OHIO, US); L. BIRKOFER et al.: "N-carboxy-beta-amino acid anhydrides and their ploymerization"**

(73) Titulaire: **RHONE- POULENC SANTE, Les Miroirs 18 Avenue d'Alsace, F-92400 Courbevoie Cedex (FR)**

(72) Inventeur: **Arnaud, Michel, 31, rue Boileau, F-69006 Lyon (FR)**
Inventeur: **Corbet, Jean- Pierre, "Les Marronniers" Résidence "Charrière Blanche", F-69130 Ecully (FR)**

(74) Mandataire: **Pilard, Jacques, RHONE- POULENC RECHERCHES Service Brevets Pharma 25, Quai Paul Doumer, F-92408 Courbevoie Cedex (FR)**

EP 0 143 018 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne un nouveau procédé de préparation de tétrahydro-1, 2, 3, 4 quinolinones-4 de formule générale:

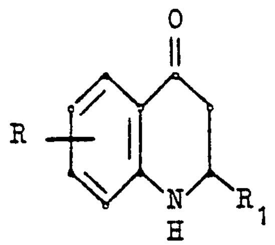

(I)

qui sont particulièrement utiles comme intermédiaires dans la synthèse de substaces thérapeutiquement actives.

Dans la formule générale (I), R représente un atome d'hydrogène ou un substituant choisi dans le groupe des atomes d'halogène et des radicaux alcoyles droits ou ramifiés contenant 1 à 4 atomes de carbone et alcoyloxy droits ou ramifiés contenant 1 à 4 atomes de carbone, et $R_1$ réprésente un atome d'hydrogène ou un radical alcoyle droit ou ramifié contenant 1 à 4 atomes de carbone.

Il est connu de préparer des tétrahydro-1, 2,3,4 quinolinones-4 de formule générale (I) par cyclisation d'un acide anilino-3 propionique au moyen d'acide polyphosphorique selon le procédé décrit dans le brevet français FR 1 514 280, au moyen d'un oléum selon le procédé décrit dans le brevet européen EP 56 764 ou encore au moyen d'un mélange acide fluorhydrique-trifluorure de bore selon le procédé décrit dans le brevet européen EP 56 763.

Il faut cependant remarquer que, dans le cas particulier de la cyclisation de l'acide m.chloroanilino-3 propionique par l'acide polyphosphorique, un mélange en quantités sensiblement égales de chloro-5 et de chloro-7 tétrahydro-1,2,3,4 quinolinone -4 est obtenu. La sélectivité en chloro-7 tétrahydro-1,2,3,4 quinolinone-4 est notablement améliorée si on utilise comme agent de cyclisation un oléum ou un mélange acide fluorhydrique-trifluorure de bore mais la mise en oeuvre industrielle de ces procédés se heurte à des difficultés résultant soit de la manipulation de quantités importantes d'acide sulfurique soit de l'utilisation d'un mélange d'acide fluorhydrique anhydre et de trifluorure de bore.

Il a maintenant été trouvé, et c'est ce qui fait l'objet de la présente invention, que les produits de formule générale (I) peuvent être obtenus avec de bons rendements et, lorsque le cas se présente, avec une sélectivité accrue, par cyclisation intramoléculaire d'un produit de formule générale:

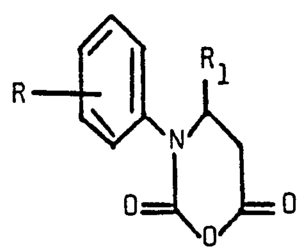

(II)

dans laquelle R et $R_1$ sont définis comme précédemment, au moyen d'un acide de Lewis ou d'un acide fort dans un solvant convenable.

Comme acides de Lewis conviennent particulièrement bien le chlorure d'aluminium, le chlorure ferrique, le chlorure de titane ou le chlorure stannique.

Comme acides forts conviennent particulièrement bien l'acide sulfurique, l'acide fluorhydrique, les acides sulfoniques tels que l'acide méthanesulfonique, l'acide polyphosphorique, l'acide trichloroacétique ou l'acide trifluoroacétique.

Lorsque l'on utilise un acide de Lewis la réaction est mise en oeuvre dans un solvant organique convenablement choisi à une température comprise entre 20 et 60° C. Comme solvants organiques peuvent être utilisés les hydrocarbures halogénés tels que le chlorure de méthylène et le trichloro-1,1,2 éthane, le sulfure de carbone, les nitroalcanes ou le tétrachloroéthylène.

Lorsque l'on utilise un acide fort, la cyclisation s'effectue par chaffage à une temperature comprise entre 20 et 100° C.

Le produit de formule générale (I) peut être isolé du mélange réactionnel et purifié par application des méthodes habituelles.

Le produit de formule générale (II) peut être obtenu à partir d'un acide anilino-3 propionique de formule générale:

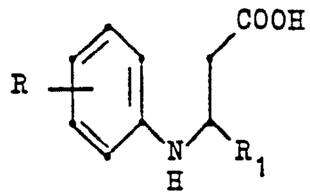

(III)

dans laquelle R et $R_1$ sont définis comme precedemment, qui est traité par le phosgène pour obtenir un produit de formule générale:

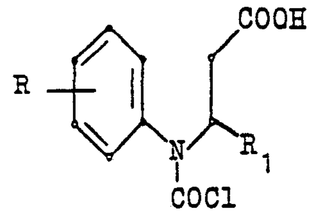

(IV)

dans laquelle R et $R_1$ sont définis comme précédemment, qui sous l'action d'une base ou par chauffage est transformé en produit de formule générale:

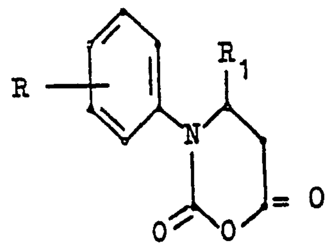

(II)

dans laquelle R et $R_1$ sont définis comme précédemment en opérant dans les conditiona décrites par L. Biekofer et R. Modic, Ann., 604, 56-62 (1957).

Les produits de formule génériae (IV) sont obtenus par action du phosgène sur un produit de formule générale (III) en opérant dans un solvant organique tel que le chlorure de méthylène, le trichloro-1,1,2 éthane, les esters aliphatiques ou le dioxanne à une température comprise entre -10 et 150°C et de préférence entre 30 et 80°C. Généralement, on utilise un léger excés de phosgène (inférieur à 10 %) par rapport à l'acide anilino-3 propionique de formule générale (III) mis en oeuvre. Il est avantageux d'opérer en atmosphère inerte.

Les produits de formule générale (II) peuvent être obtenus par action d'une base telle que la triéthylamine ou la pyridine sur un produit de formule générale (IV) ou par simple chauffage d'un produit de formule générale (IV) à une température supérieure à 50°C et de préférence entre 70 et 150°C, dans un solvant organique anhydre choisi parmi les éthers, les esters aliphatiques, comme l'acétate d'isopropyle et les solvants chlorés aliphatiques comme le trichloro-1,1,2 éthane.

Les acides de formule générale (III) utilités comme produits de départ peuvent être obtenius par action d'une aniline convenablement substituée sur un acide de formule générale:

$R_1$-CH=CH-COOH (V)

dans laquelle $R_1$ est défini comme précédemment.

La réaction s'effectue généralement dans l'eau à une température comprise entre 70 et 100°C en utilisant un excès d'aniline par rapport à l'acide de formule générale (V) mis en oeuvre. La durée de la réaction est généralement comprise entre 1 et 4 heures.

Les tétrahydro-1,2,3,4 quinolinones-4 de formule générale (I) sont particulièrement utiles comme intermédiaires dans la synthèse de substances thérapeutiquement actives telles que la chloroquine, la glafénine, l'antrafénine ou l'amodiaquine.

Plus particulièrement, la chloro-7 tétrahydro-1,2,3,4 quinolinone-4 peut être transformée en chloroquine par condensation de la diéthylamino-4 méthyl-1 butylamine en présence d'air selon le procédé décrit par W.S. Johnson et B.G. Buell, J. Amer. Chem. Soc., 74, 4513 (1952).

Les exemples suivants, donnés à titre non limitatif, montrent comment l'invention peut être mise en pratique.

**Example 1**

Dans un erlenmeyer de 50 $cm^3$ muni d'un réfrigérant et maintenu sous atmosphère d'argon, on introduit 0,334g (1,48 m.mole) de m.chlorophényl-3 dioxo-2,6 perhydro-oxazine-1,3 en solution dans 5 $cm^3$ de chlorure de méthylène. On ajoute, en 5 minutes, à une température voisine de 20°C, 0,453 g (3,4 m.moles) de chlorure d'aluminium anhydre et 2 $cm^3$ de chlorure de méthylène. Il se forme tout d'abord une suspension blanc-jaune qui se redissout vers la fin de l'addition du chlorure d'aluminium. Après une heure d'agitation, il y a disparition du produit de départ. La réaction est arrêtée après 1 heure 30 minutes,

Le mélange réactionnel est versé sur 50 g de glace. Le pH de la phase aqueuse est compris entre 4 et 5. On extrait les produits organiques formés par trois fractions de chlorure de méthylène. La solution chlorométhylénique est lavée à l'eau puis séchée sur sulfate de sodium anhydre. Après filtration puis évaporation du filtrat, on obtient 0,258g d'un solide jaune clair.

L'analyse de ce solide par chromatographie en phase vapeur et par chromatographie liquide haute performance montre qu'il contient 95 à 96 % de chloro-7 tétrahydro-1,2,3,4 quinolinone-4 et 0,5 % de chloro-5 tétrahydro-1,2,3,4 quinolinone-4.

Le rendement est de 92 % par rapport à la m.chlorophényl-3 dioxo-2,6 perhydro-oxazine-1,3 mise en oeuvre.

La m-chlorophényl-3 dioxo-2,6 perhydro-oxaline-1,3 peut être préparée de la manière suivante:

A un mélange de 510,3 g de m.chloroaniline dans 150 $cm^3$ d'eau, maintenu sous atmosphère d'argon agité à 80°C, on ajoute en 10 minutes une solution de 72,05 g d'acide acrylique dans 100 $cm^3$ d'eau. Le mélange réactionnel, constitué de deux phases, est maintenu pendant 3 heures à 80°C sous agitation puis refroidi à 20°C. Aprés décantation, la phase aqueuse (couche supérieure) est éliminée. A la phase organique on ajoute 423 $cm^3$ d'une solution aqueuse de soude 2,6 N, en agitant et en maintenant la température à 20°C. Après décantation, la phase organique constituée de 303 g de m.chloroaniline est séparée. La phase aqueuse (850 $cm^3$) est extraite 6 fois successivement par 450 $cm^3$ d'éther.

La phase aqueuse, dont on élimine l'éther par évaporation sous pression réduite (20 mm de mercure; 2,7 kPa), est acidifiée par addition de 105 g d'acide sulfurique à 50 % (en poids). Le pH final est 3,5 (point isoélectrique). La température passe de 22 à 33°C puis on chauffe à 40°C. Après décantation, on sépare:

- une phase organique inférieure (208,8 g) constituée d'acide m.chloroanilino-3 propionique fondu saturé d'eau (8,6 % d'eau)
- une phase aqueuse supérieure (601 g) contenant 2,28 g d'acide m.chloroanilino-3 propionique et 156 g de sulfate de sodium.

La phase organique est chauffée pendant 1 heure à 80°C sous pression réduite (20 mm de mercure; 2,7 kPa). On obtient 195,4 g d'un produit contenant 94 % d'acide m.chloroailino-3 propionique et 2,3 % d'eau.

Dans un ballon de 100 $cm^3$ muni d'une agitation magnétique, d'un thermomètre, d'un réfrigérant ascendant à acétone-carboglace, d'un dispositif d'introduction de gaz et d'une ampoule de coulée, on introduit 20 $cm^3$ de chlorue de méthylène sec. On condense, à une température comprise entre 0 et 5°C, 3,15 g (31,8 m.moles) de phosgène. Le mélage réactionnel est maintenu sous atmosphère d'argon. On ajoute ensuite, en 19 minutes, une solution de 6,25 g (31,3 m.moles) d'acide m.chloroanilino-3 propionique dans 15 $cm^3$ de chloruie de méthylène. La température monte de 6 à 23°C. Le mélage réactionnel est alors constitué d'une phase liquide jaune et d'un précipité blanc en suspension. On poursuit l'agitation pendant 10 minutes à 23°C puis on fait passer, pendant 50 minutes, un courant d'argon dans le mélange réactionnel pour éliminer le phosgène n'ayant pas réagi.

Le précipité est séparé par filtration sous courant d'argon puis séché sous pression réduite (1mm de mercure; 0,13 kPa) jusqu' à poids constant. On obtient ainsi 3,2 g de chlorhydrate de l'acide m.chloroanilino-3 propionique.

Le filtrat est concentré à sec. On obtient ainsi 4,58 g d'acide N-chloroformyl m.chloroanilino-3 propionique, pratiquement pur, fondant à 106°C dont la structure est confirmée par le spectre infra-rouge, le spectre de masse et le spectre de résonace magnétique nucléaire du proton. Le produit, après recristallisation dans l'éther isopropylique, fond à 111°C.

Dans un ballon de 50 $cm^3$ muni d'une agitation magnétique, d'un réfrigérant à eau et d'une ampoule de coulée, on introduit, sous atmosphère d'argon, 25 $cm^3$ d'éther éthylique sec puis 1,06 g (4,06 m.moles) d'acide N-chloroformyl m.chloroanilino-3 propionique. On ajoute ensuite en 5 minutes à une température voisine de 20°C une solution de 0,6 $cm^3$ (4,3 m.moles) de triéthylamine dans 2 $cm^3$ d' éther éthylique sec. Il se forme un précipité blanc abondant. Après 1 heure 15 minutes de contact, il ne subsiste plus de produit de départ par chromatographie sur couche mince. Après filtration et séchage à poids constant du précipité, on obtient 1,322 g d'un melange constitué de chlorhydrate de triéthylamine et de m.chlorophényl-3 dioxo-2,6 perhydro-oxazine-1,3. On met en suspension 1,138 g du précipité obtenu dans environ 20 $cm^3$ d'acétate d'éthyle sec. Après filtration le filtrat est concentré à sec.

On obtient ainsi 0,676 g de m.chlorophényl-3 dioxo-2,6 perhydro-oxazine-1,3 sous forme d'un solide blanc fondant à 126°C et dont la structure est confirmée par le spectre infra-rouge, le spectre de masse et le spectre de résonance magnétique nucléaire du proton.

**Example 2**

Dans un ballon de 20 $cm^3$ muni d'une agitation magnétique d'un thermomètre, d'un réfrigérant et d'une ampoule de coulée on introduit 0,514 g (2,27 m.moles) de m.chlorophényl-3 dioxo-2,6 perhydro-oxazine-1,3 en solution dans 15 $cm^3$ de chlorure de méthylène. On ajoute, en une fois à une température voisine de 20° C, 0,371 g (2,28 m.moles) de chlorure ferrique. Le mélange réactionnel est maintenu sous agitation pendant 28 heures à 40° C. Après refroidissement à une température voisine de 20° C et addition de 5 $cm^3$ d'acide chlorhydrique N et de 20 $cm^3$ d'eau distillée, la phase organique est séparée puis lavée par de l'eau distillée. La solution chlorométhylénique est alors séchée sur sulfate de sodium anhydre. Après filtration puis évaporation du solvant, on obtient 0,329 g d'un résidu orangé.

L'analyse de ce résidu par chromatographie en phase vapeur montre qu'il contient 33 % de chloro-7 tétrahydro-1,2,3,4 quinolinone-4 et moins de 1 % de chloro-5 tétrahydro-1,2,3,4 quinolinone-4.

Le rendement est de 26 % par rapport à la m-chlorophényl-3 dioxo-2,6 perhydro-oxazine-1,3 mise en oeuvre.

**Example 3**

Dans un ballon de 20 $cm^3$ muni d'une agitation magnétique, d'un thermomètre, d'un réfrigérant et d'une ampoule de coulée on introduit 0,224 g (0,990 m.moles) de m-chlorophényl-3 dioxo-2,6 perhydro-oxazine-1,3 en solution dans 5 $cm^3$ de chlorure de méthylène anhydre. On ajoute, en une fois, à une température voisine de 20° C, 0,189 g (0,910 m.moles) de tétrachlorure de titane. Le mélange réactionnel est maintenu sous agitation pendant 18 heures à une température voisine de 40° C. On ajoute alors 5 $cm^3$ de dichloro-1,2 éthane et élimine le chlorure de méthylène par distillation. Le mélange réactionnel est ensuite maintenu sous agitation pendant 54 heures 15 minutes à une température voisine de 80° C. Après refroidissement, le mélange réactionnel est traité d'une manière analogue à celle décrite dans l'exemple 4. On obtient 0,172 g d'un résidu huileux.

L'analyse de ce résidu par chromatographie en phase gazeuse montre qu'il contient 34 % de chloro-7 tétrahydro-1,2,3,4 quinolinone-4 et moins de 1 % de chloro-5 quinolinone-4.

Le rendement est de 31 % par rapport à la m-chlorophényl-3 dioxo-2,6 perhydrooxazine-1,3 mise en oeuvre.

**Example 4**

En opérant d'une maniére analogue à celle décrite à l'exemple 1 mais à partir de 1,06 9 (5,20 m.moles) de dioxo-2,6 (méthyl-2 phényl)-3 perhydro-oxazine-1,3, de 17 $cm^3$ de chlorure de méthylène et de 1,70 g de chlorure d'aluminium on obtient 0,84 g d'un solide jaune clair fondant entre 92°C et 94° C. L'analyse de ce produit par spectrométrie infrarouge, de masse et de résonance magnétique nucléaire, montre qu' il s' agit de méthyl-8 tétrahydro-1,2,3,4 quinolinone-4 pratiquement pure. Le produit, après recristallisation dans l'hexane, fond à 98° C.

La dioxo-2,6 (méthyl-2 phényl)-3 perhydro-oxazine-1,3 peut être préparée de la manière suivante:

On prépare l'acide (méthyl-2 anilino)-3 propionique d'une manière analogue à celle décrite dans l'exemple 1 mais à partir de 64,3 g (0,60 mole) d'o-toluidine, de 11,6 g (0,16 moles) d'acide acrylique et de 36 $cm^3$ d'eau.

On obtient 23,85 g d'une poudre blanche qui, après recristallisation dans un mélange toluène-hexane 70-30 (en volumes), donne 21,7 g (0,12 mole) de produit pur fondant à 87° C.

Dans un ballon de 100 $cm^3$ muni d'une agitation magnétique, d'un thermomètre, d'un réfrigérant ascendant à carbo-glace-acétone, d'un dispositif d'introduction de gaz et d'une ampoule de coulée on introduit 5,29 g (29,6 m.moles) d'acide (méthyl-2 anilino)-3 propionique et 30 $cm^3$ de chlorure de méthylène. Le mélange maintenu sous atmosphère d'argon, est chauffé à une température voisine de 40° C puis on ajoute, sous agitation, en 25 minutes, 5,20 g (52,6 m.moles) de phosgène en maintenant le température entre 36°C et 40°C. Pendant l'introduction, il y a formation d'un précipité blanc de chlorhydrate de l'acide (méthyl-2 anilino)-3 propionique. Le mélange réactionnel est chauffé au voisinage de 40°C pendant encore une heure. Le précipité blanc est alors pratiquement dissous en totalité. Après refroidissement à 20° C et élimination du phosgène n'ayant pas réagi, le léger precipité restant est séparé par filtration. Le filtrat est concentré à sec. On obtient ainsi 7 g d'acide N-chloroformyl (méthyl-2 anilino)-3 propionique pratiquement pur, fondant à 112°C dont la structure est confirmée par le spectre infra-rouge, le spectre de masse et le spectre de résonance magnétique nucléaire du proton. Le produit, après recristallisation dans un mélange toluène-hexane 70-30 (en volumes), fond à 113° C.

En opérant d'une manière analogue à celle décrite à l'exemple 2, mais à partir de 2,42 g (10,0 m.moles) d'acide N-chloroformyl (méthyl-2 anilino)-3 propionique, de 40 $cm^3$ d'éther éthylique sec et de 1,52 $cm^3$ de triéthylamine, on obtient 1,25 g d'un résidu semi-solide blanc constitué de dioxo-2,6 (méthyl-2 phényl)-3 perhydrooxazine-1,3 pratiquement pure.

Le produit, après recristallisation dans une mélange hexaneacétate d'éthyle 50-50 (en volumes), fond entre 165° C et 170° C.

La structure est confirmée par le spectre infra-rouge, le spectre de masse et le spectre de résonance magnétique nucléaire du proton.

**Example 5**

En opérant d'une manière analogue à celle décrite à l'exemple 1 mais à partir de 0,65 g (2,9 m.moles) de dioxo-2,6 (méthoxy-4 phényl)-3 perhydrooxazine-1,3, de 15 $cm^3$ de chlorure de méthylène et de 0,79 g (5,9 m.moles) de chlorure d'aluminium, on obtient 0,44 g d'un solide jaune fondant à 110°C. L'analyse de ce produit par spectrométrie infra-rouge, de masse et de résonance magnétique nucléaire montre qu'il s'agit de méthoxy-6 tétrahydro-1,2,3,4 quinolinone-4 pratiquement pure. Après recristallisation dans le toluène, le produit fond à 111°C.

La dioxo-2,6 (méthoxy-4 phényl)-3 perhydrooxazine-1,3 peut être préparée de la manière suivante:

On prépare l'acide (méthoxy-4 anilino)-3 propionique d'une manière analogue à celle décrite dans l'exemple 1 mais à partir de 61 g (0,495 moles) de p.anisidine, de 9,5 g (0,132 mole) d'acide acrylique et de 12 $cm^3$ d'eau. On obtient, après recristallisation dans le toluène, 11,7 g (0,060 mole) de produit pur, sous forme d'une poudre blanche, fondant à 89°C.

En opérant d'une manière analogue à celle décrite à l'exemple 4, mais à partir de 2,88 g (14,7 m.moles) d'acide (méthoxy-4 anilino)-3 propionique, de 20 $cm^3$ de chlorure de méthylène et de 4,3 g (43,5 m.moles) de phosgène, on obtient 3,77 g d'acide N-chloroformyl (méthoxy-4 anilino)-3 propionique pratiquement pur sous forme d'une poudre blanche fondant à 90°C. Après recristallisation dans un mélange hexane-acétate d'éthyle 50-50 en volumes le produit fond à 93°C. La structure du produit obtenu est confirmée par le spectre infra-rouge, le spectre de masse et le spectre de résonance magnétique nucléaire du proton.

En opérant d'une manière analogue à celle décrite à l'exemple 1 mais à partir de 2,54 g (9,9 m.moles) d'acide N-chloroformyl (méthoxy-4 anilino)-3 propionique, de 40 $cm^3$ d'éther éthylique sec, et de 1,52 $cm^3$ de triéthylamine, on obtient 1,19 g d'une poudre blanche fondant vers 140°C. L'analyse de ce produit par spectrométrie infra-rouge, de masse et de résonance magnétique nucléaire montre qu'il s'agit de dioxo-2,6 (méthoxy-4 phényl)-3 perhydro-oxazine-1,3 presque pure. Le produit, après recristallisation dans un mélange hexane-acétate d'éthyle 30-70 (en volumes), fond vers 144°C.

**Revendications**

- Procédé de Préparation d'une tétrahydro-1,2,3,4 quinolinone-4 de formule générale:

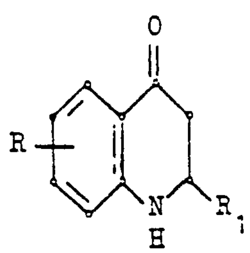

dans laquelle R représente un atome d'hydrogène ou un substituant choisi dans le groupe des atomes d'halogène et des radicaux alcoyles droits ou ramifiés contenat 1 à 4 atomes de carbone et alcoyloxy droits ou ramifiés contenant 1 à 4 atomes de carbone et $R_1$ représente un atome d'hydrogène ou un radical alcoyle droit ou ramifié contenant 1 à 4 atomes de carbone caractérisé en ce que l'on traite un produit de formule générale:

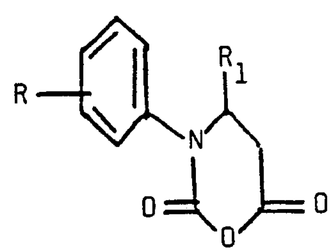

dans laquelle R et $R_1$ sont définis comme précédemment par un acide de Lewis choisi parmi le chlorure d'aluminium, le chlorure de titane, le chlorure stannique ou le chlorure ferrique dans un solvant organique choisi parmi les hydrocarbures halogénés, le sulfure de carbone, les nitroalcanes et le tétrachloroéthylène, ou par un acide fort choisi parmi l'acide sulfurique, l'acide fluorhydrique, les acides sulfoniques, l'acide polyphosphorique, l'acide trichloroacétique ou l'acide trifluoroacétique.

**Patentansprüche**

Verfahren zur Herstellung eines 1,2,3,4-Tetrahydro-chinolin-4-ons der allgemeinen Formel:

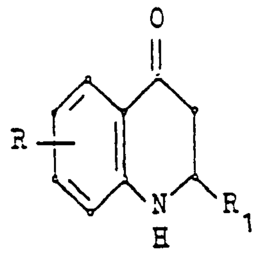

in welcher R ein Wasserstoffatom oder einen Substituenten, ausgewählt aus der Gruppe der Halogenatome und der geradkettigen oder verzweigten Alkylreste mit 1 bis 4 Kohlenstoffatomen und der geradkettigen oder verzweigten Alkoxyreste mit 1 bis 4 Kohlenstoffatomen darstellt und $R_1$ ein Wasserstoffatom oder einen geradkettigen oder verzweigten Alkylrest mit 1 bis 4 Kohlenstoffatomen darstellt, dadurch gekennzeichnet, daß man eine Verbindung der allgemeinen Formel

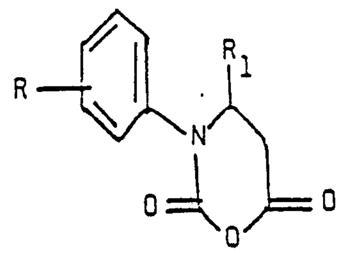

in welcher R und $R_1$ die obige Bedeutung haben, mit einer Lewis-Säure, ausgewählt aus Aluminiumchlorid, Titanchlorid, Zinnchlorid oder Eisen(III)-chlorid in einem organischen Lösungsmittel, ausgewählt aus den Halogenkohlenwasserstoffen, Schwefelkohlenstoff, den Nitroalkanen und Tetrachloräthylen, oder mit einer starken Säure, ausgewählt aus Schwefelsäure, Fluorwasserstoffsäure, den Sulfonsäuren, Polyphosphorsäure, Trichloressigsäure oder Trifluoressigsäure, behandelt.

**Claims**

Process for preparing a 1,2,3,4-tetrahydro-4-quinolinone of general formula:

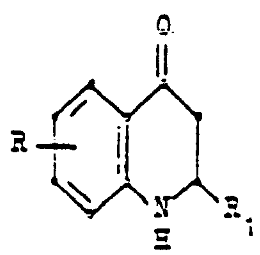

in which R denotes a hydrogen atom or a substituent chosen from the group consisting of halogen atoms, linear or branched alkyl radicals containing 1 to 4 carbon atoms and linear or branched alkoxy radicals containing 1 to 4 carbon atoms, and $R_1$ denotes a hydrogen atom or a linear or branched alkyl radical containing 1 to 4 carbon atoms, characterized in that a product of general formula:

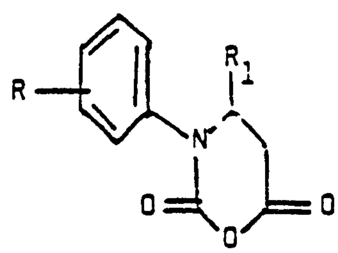

in which R and $R_1$ are defined as above, is treated with a Lewis acid chosen from aluminium chloride, titanium chloride, stannic chloride or ferric chloride in an organic solvent chosen from halogenated

hydrocarbons, carbon disulphide, nitroalkanes and tetrachloroethylene, or with a strong acid chosen from sulphuric acid, hydrofluoric acid, sulphonic acids, polyphosphoric acid, trichloroacetic acid or trifluoroacetic acid.